## Europäisches Patentamt
## European Patent Office
## Office européen des brevets

(19)

(11) Publication number: **0 079 093**
**B1**

(12) **EUROPEAN PATENT SPECIFICATION**

(45) Date of publication of patent specification: **11.06.86**

(51) Int. Cl.[4]: **C 07 C 85/24, C 07 C 87/50**

(21) Application number: **82201301.7**

(22) Date of filing: **18.10.82**

(54) Process for the selective alkylation of an aniline.

(30) Priority: **11.11.81 GB 8133996**

(43) Date of publication of application:
**18.05.83 Bulletin 83/20**

(45) Publication of the grant of the patent:
**11.06.86 Bulletin 86/24**

(84) Designated Contracting States:
**BE CH DE FR GB IT LI NL SE**

(56) References cited:
**DE-A-1 051 271**
**FR-A- 815 532**
**GB-A-2 064 537**
**US-A-2 092 972**

**ORGANIC REACTIONS, vol. III, pages 1-82,
John Wiley & Sons, Inc., New York (USA); C.C.
PRICE: "The alkylation of aromatic compounds
by the Friedel-Crafts method"**

(73) Proprietor: **SHELL INTERNATIONALE
RESEARCH MAATSCHAPPIJ B.V.
Carel van Bylandtlaan 30
NL-2596 HR Den Haag (NL)**

(72) Inventor: **Baardman, Frank
Badhuisweg 3
NL-1031 CM Amsterdam (NL)**
Inventor: **van Helden, Robert
Badhuisweg 3
NL-1031 CM Amsterdam (NL)**
Inventor: **de Nie-Sarink, Margaretha Johanna
Badhuisweg 3
NL-1031 CM Amsterdam (NL)**

(74) Representative: **Hunter, Keith Roger Ian et al
4 York Road
London SE1 7NA (GB)**

Courier Press, Leamington Spa, England.

## Description

This invention relates to a process for the selective alkylation of an aniline.

It is well known that aromatic compounds can be alkylated in the aromatic nucleus by reaction with various alkylating agents in the presence of a protonic acid or a Lewis acid as catalyst. This is the Friedel-Crafts reaction. A very wide range of acidic catalysts, of which aluminium chloride may be the best-known example, has been used to prepare a wide variety of alkyl-substituted benzene derivatives.

Such reactions are of great importance and of wide applicability. They can however suffer from at least three principle limitations. First, it can be rather difficult to prevent dialkylation of the benzene nucleus, since the first product formed, the monoalkylated product, tends to be more reactive than that starting material. Thus it is often necessary to conduct the reaction in such a way that only a relatively small proportion of the aromatic starting material reacts, and then to separate off the product or products formed, and recycle the starting material. On an industrial scale, such procedures can be very costly.

Secondly, when using certain substituted benzenes as starting materials, alkyl substitution can lead to two or more positional isomers. In some cases, one isomer is formed in large excess over the other possible isomers; in many cases however the reaction is relatively unspecific, and mixtures of some or all of the possible isomers are formed.

Thirdly as is stated clearly in a well known university textbook (R. T. Morrison and R. N. Boyd, *Organic Chemistry, third edition*, (Boston) 1974, page 382, Chapter 12.8, "Limitations of Friedel-Crafts alkylation")," ... aromatic rings containing the —$NH_2$,—$NHR$, or $NR_2$ group do not undergo Friedel-Crafts alkylation, partly because the strongly basic nitrogen ties up the Lewis acid needed for ionization of the alkyl halide ... tying up of the acidic catalyst by the basic nitrogen is not the only factor that prevents alkylation, since even when excess catalyst is used, reaction does not occur". Only one exception to this general rule has been discovered, in 1955, by Stroh et al (German Auslegeschrift 1051271), who found that it is possible to alkylate aromatic amines using liquified lower olefins in the presence of e.g. $AlCl_3$, at high temperatures and pressures, e.g. 300°C and 250 atm. Under these severe conditions the aromatic nucleus is alkylated, selectively, but only at the position(s) ortho to the amino group. Only if both ortho positions are already occupied by substituents, and then not always, does para-alkylation take place. The highest molar ratio of aluminium halide to the aniline used is 0.028.

Further, from Houben-Weyl, Volume XI/1 (1957) pages 1019—1023 and 848—849 it is known that alkylation of anilines requiring rather severe conditions, tends to yield N-alkyl derivatives which at high temperatures (200—300°C) are rearranged to give derivatives having the alkyl substituent on the ortho or para position in the aromatic nucleus.

Another high temperature process is described in FR—A—815 532, disclosing the alkylation of anilines with $C_{10-18}$ alcohols over a zinc or cobalt halide catalyst at a temperature of at least 200°C, yielding p-alkyl aniline derivatives.

In US—A—2 092 972 the alkylation of N-acyl anilines over aluminium halides is described at temperatures below 50°C. It is, however, acknowledged that no successful alkylation of anilines was known so far.

Hence, no process is known in which 4-alkylanilines can be prepared by nuclear alkylation of anilines with reasonable yield and selectivity to the desired product (selective alkylation) at low temperatures.

The invention therefore provides a process for the selective alkylation (as hereinafter defined) of an aniline containing an —$N(R)_2$ group in which each R independently represents a hydrogen atom or an alkyl group, by reacting the aniline with an alkylating agent in the presence of an molar excess of an aluminium halide, characterised in that the aniline is selectively alkylated at the nuclear position para to the amino group by reacting it in a polar organic solvent at a temperature in the range from −30° to +40°C in the presence of at least 1.02 mole of aluminium halide per mole of the aniline.

A considerable advantage of the present process in comparison to the known method is that the reaction does not have to be carried out at high temperatures or pressures. The alkylation is carried out at a temperature in the range from −30 to 40°C, preferably in the range from −5 to 20°C, in particular from 5 to 10°C. If the volatilities of the reagents at the reaction temperature chosen do not necessitate the use of pressure vessels, it is generally convenient if the alkylation is carried out at atmospheric pressure.

The reaction should be carried out in the liquid phase in the presence of a polar solvent. The solvent is preferably an organic liquid, sufficiently polar to dissolve the aluminium halide-aniline complex, and also able to dissolve sufficient of the alkylating agent. It is preferably anhydrous. Very suitable as solvent is 1,2-dichloroethane, but other chlorinated aliphatic lower hydrocarbons such as methylene chloride may also be used. Sometimes the solvent and the alkylating agent may be the same compound, especially if the present alkylation process is used for the preparation of polyalkylated anilines.

The catalyst used is an aluminium halide, e.g. aluminium bromide and/or aluminium chloride. Aluminium chloride is preferred.

On contacting the aluminium halide and the aniline a 1:1 complex is formed, usually accompanied by a temperature rise. It is possible to dissolve the aniline in a suitable quantity of solvent before adding the aluminium halide, but it is equally possible to introduce the aluminium halide first and then to add the aniline. The presence of a solvent has the added advantage of absorbing some of the reaction heat. There is a distinct lower limit of 1.02 equivalents of aluminium halide relative to the aniline; however, there is no distinct upper limit. Preferably from 1.05 to 2.0 mole aluminium halide per mole of the aniline is used, in particular from 1.05 to 1.15 mole/mole, especially 1.07 mole/mole.

As alkylating agent many of the known Friedel-Crafts alkylating agents are suitable. Preferably an alkyl halide or an alkanol is used as the alkylating agent. However, when using an alkanol it should be remembered that the aluminium halide may complex with it, so that an extra equivalent (based on the alkanol) of catalyst should be added. The water formed when using alkanols as alkylating agents can have a detrimental effect on the catalyst, which may be reflected in the conversion and the selectivity figures. Alkyl halides, especially alkyl chlorides, are therefore preferred as alkylating agents.

The group engaged in the alkylation as defined in this application may for example be a lower alkyl group, for example methyl, ethyl, propyl, isopropyl, butyl, isobutyl, sec. butyl, and tert. butyl, but larger groups, e.g. $C_{30}H_{61}$, are not excluded. The group may contain one or more double or triple bonds, and it may contain saturated or aromatic ring structures. Some of the hydrogen atoms of the group may be replaced by a substituent, for example a halogen atom or an hydroxyl group. Should a substituent complex with the aluminium halide catalyst, as is the case with the hydroxyl group, then it is sufficient to add an extra equivalent of catalyst allowing for this effect.

As is normal in Friedel-Crafts reactions, a group introduced into the aniline nucleus may in some cases be a different group from a group present in the alkylating agent. Usually, products in which the group introduced is a tertiary or secondary alkyl group rather than a primary group, predominate when the group has 3 or more carbon atoms. For example, an isopropyl group can be introduced by the use of either isopropyl chloride or n-propyl chloride as alkylating agent: under the reaction conditions, the same transient intermediate is formed and leads mainly to isopropylation of the aniline nucleus. The use of isopropyl chloride, however, causes a faster conversion.

Olefins and dialkyl ethers may also be used as alkylating agents.

Whatever alkylating agent is selected, it is in general preferred that the alkylating agent is added quickly, preferably all in once, to the aniline and the aluminium halide. It appears that straight addition, especially when working without added solvent, provides high conversions and high selectivities to para-substitution. The above refers of course to batch preparations, but it is to be understood that the present process can be carried out continuously as well. In the latter case a plug flow type reactor is preferred, resulting in a narrow residence time distribution of the reactants, and in which the residence time can be varied according to the required product purity and yield.

Analyses of the product after regular periods of time from the start of the reaction indicate that the conversion of the aniline increases with time, but that the selection to para-substituted product tends to decrease. To prevent further alkylation, it is advantageous if the alkylation reaction is stopped by adding a quenching agent.

Preferably the quenching agent destroys the catalyst and/or the complex between the catalyst and the aniline: e.g. water or alcohol could be used. It appears to be particularly advantageous however, if the quenching is combined with the working-up for the product. For example, when using an alkyl chloride as alkylating agent, it may be desirable that the HCl formed be neutralized and/or removed from the amino group of the aniline. In this case a suitable quenching agent is an aqueous solution of a base, e.g. caustic soda or ammonia.

The molar ratio of the alkylating agent to the aniline is not critical, but preferably lies in the range of from 0.5 to 1.30, in particular from 0.8 to 1.2. It appears that at low molar ratios the conversion of the aniline is low, but that the selectivity toward para-substitution is high, whereas at higher molar ratios this situation reverses.

If the present alkylation is applied in the preparation of polyalkylated products—including a para-alkyl group—the alkylation reaction should be allowed to proceed as long as practicable and/or necessary. The more alkylating agent is used, the more polyalkylated products will be formed.

The aniline may be any aromatic amine, including aniline $C_6H_5NH_2$ itself. The amino group may be primary, secondary or tertiary—i.e. the compound may contain the $NH_2$ group or may be an N-alkyl or N,N-dialkyl compound. The aromatic nucleus of the aniline may be substituted, except of course at the para-position, with one or more substituents, such as halo, nitro, nitroso, acyl, alkoxy, sulphonyl, hydroxy and cyano groups, and in particular alkyl groups. Certain substituents however are known to complex with the aluminium halide, in which case one or more extra equivalents of aluminium halide should be added to allow for this effect. Advantageously the aniline is a nuclear alkylated aniline, especially 3-methylaniline (m-toluidine).

Under optimum conditions, conversions of about 80% and selectivities to the para-compound of 70—80% can be achieved when using m-toluidine as the aniline and isopropyl chloride as the alkylating agent. Advantageously the alkylating agent is isopropyl chloride, if one wants to prepare 3-methyl-4-isopropyl-aniline. This compound is an intermediate in the preparation of 1,1-dimethyl-3-(4-isopropyl-3-methyl-phenyl)urea, which is an extremely potent selective herbicide for the control of weeds in ceral crops. Thus the invention also provides a process for the preparation of said urea, which process includes the step of preparing the compound 3-methyl-4-isopropylaniline by the process according to the invention. Conversion of the aniline to the urea may for example be carried out by reaction of the aniline with a dimethylcarbamoyl halide, or by reaction of the aniline with phosgene followed by reaction with dimethylamine.

The following Examples illustrate the invention. Analyses were carried out by NMR and/or gas-liquid chromatography. The results of examples 1 to 9 are tabulated in table I.

Example 1

Analytical quality 97% pure aluminium chloride (13.73 g; 99.9 mmol) was added to a stirred solution of m-toluidine (10.0 g; 93.9 mmol) in dried 1,2-dichloroethane (100 ml) at room temperature, under nitrogen. The molar ratio of $AlCl_3$ to m-toluidine thus amounted to 1.07:1. The temperature rose to about 45°C. The reaction mixture was kept at this temperature until all $AlCl_3$ had dissolved. The resulting clear, dark-brown solution was cooled to 10°C in a thermostatted water bath. Subsequently, a solution of isopropyl chloride (10.0 ml; 110 mmol) in 1,2-dichloroethane (30 ml) was dosed to the reaction mixture in 8 minutes. The molar ratio of isopropyl chloride to m-toluidine thus amounted to 1.18:1. The temperature temporarily rose to about 12°C. A sample, taken 2 minutes after complete addition of isopropyl chloride, was shown to have composition 1A (table I).

The reaction was quenched 37 minutes after complete addition of isopropyl chloride, by the addition of 25% aqueous $NH_4OH$ (45 ml). This solution was added at such a rate that the temperature of the reaction mixture was kept below about 40°C. A massive white precipitate was formed. After complete addition of the aqueous $NH_4OH$ the white solids were filtered off and washed with diethyl ether (100 ml). The filtrate was washed with water (50 ml) and the solvent was evaporated. The residual brown oil (14.9 g) was shown to have composition 1B (table I).

Example 2

The procedure of example 1 was followed except that 13.10 g (95.3 mmol) aluminium chloride was applied. The molar ratio of $AlCl_3$ to m-toluidine thus amounted to 1.02:1. A sample taken 2 minutes to after complete addition of isopropyl chloride, was shown to have composition 2A. The product (13.1 g) was shown to have composition 2B.

Comparative Example

The experiment was repeated using only 1.01 equivalents of aluminium chloride. The solution of $AlCl_3$ and m-toluidine was green and did not turn dark brown. No heat was evolved upon addition of isopropyl chloride. No reaction occurred.

Example 3

The procedure of example 1 was followed except that the reaction was carried out at −5°C. A sample taken 2 minutes after complete addition of isopropyl chloride, was shown to have composition 3A. The product (11.7 g) was shown to have composition 3B).

Example 4

The procedure of example 1 was followed except that the reaction was carried out at 20°C. A sample taken 2 minutes after complete addition of isopropyl chloride, was shown to have composition 4A. The product (11.8 g) was shown to have composition 4B.

Example 5 ·

The procedure of example 1 was followed except that 6 ml (66 mmol) isopropyl chloride was applied. The molar ratio of alkylating agent to aniline thus amounted to 0.71:1. A sample taken 2 minutes after complete addition of isopropyl chloride, was shown to have composition 5A. The product (13.3 g) was shown to have composition 5B.

Example 6

The procedure of example 1 was followed except that 10 ml isopropyl chloride was added all at once, without 30 ml dichloroethane. The temperature temporarily rose to about 16°C. A sample taken 2 minutes after addition of isopropyl chloride, was shown to have composition 6A. The product (14.8 g) was shown to have composition 6B.

TABLE I

| | 1A | 1B | 2A | 2B | 3A | 3B | 4A | 4B | 5A | 5B | 6A | 6B | 7A | 7B | 8A | 8B | 9A | 9B |
|---|---|---|---|---|---|---|---|---|---|---|---|---|---|---|---|---|---|---|
| m-toluidine | 22.6 | 18.2 | 34.3 | 16.3 | 27.7 | 14.0 | 23.1 | 15.4 | 33.0 | 28.3 | 14.8 | 10.7 | 67.6 | 33.2 | 50.0 | 48.3 | 36.3 | 27.7 |
| o-alkylated product (2-isopropyl-5-methylaniline) | 3.9 | 3.2 | 3.3 | 3.8 | 3.2 | 3.7 | 3.4 | 2.7 | 2.9 | 2.7 | 4.7 | 3.3 | 1.3 | 2.5 | 2.2 | 2.1 | 4.6 | 4.3 |
| m-alkylated product (3-isopropyl-5-methylaniline) | 4.3 | 4.4 | 3.5 | 4.6 | 4.3 | 4.7 | 4.6 | 7.4 | 3.8 | 4.7 | 5.0 | 7.1 | 2.0 | 4.2 | 3.0 | 3.2 | 4.8 | 5.7 |
| p-alkylated product (4-isopropyl-3-methylaniline) | 64.1 | 59.1 | 56.0 | 68.5 | 60.7 | 71.5 | 58.3 | 45.0 | 55.6 | 59.7 | 72.3 | 58.1 | 28.2 | 51.9 | 41.7 | 44.5 | 48.4 | 48.0 |
| dialkylated products | 4.3 | 14.5 | 1.7 | 3.6 | 0.9 | 3.1 | 6.1 | 22.8 | 1.4 | 3.5 | 2.3 | 16.5 | 0.4 | 3.9 | 1.0 | 0.5 | 4.8 | 11.0 |

Note: all figures are GLC area percentages.

## Example 7

The procedure of example 1 was followed except that n-propyl chloride (10 ml; 114 mmol) was applied. The temperature of the reaction mixture did not change upon addition of the alkylating agent. A sample taken 2 minutes after complete addition of n-propyl chloride, was shown to have composition 7A. The product was shown to have composition 7B.

## Example 8

The procedure of example 1 was followed except that a complex of isopropyl alcohol (56 g; 93 mmol) and aluminium chloride (12.5 g of 97% purity; 91 mmol) in 1,2-dichloroethane (50 ml) was applied as the alkylating agent. The temperature temporarily rose to about 15°C. A sample taken 2 minutes after complete addition of the alkylating mixture, was shown to have composition 8A. The product (12.8 g) was shown to have composition 8B.

## Example 9

The procedure of example 1 was followed except that dried dichloromethane (250 ml) was applied as the solvent. A sample taken 2 minutes after complete addition of isopropyl chloride, was shown to have composition 9A. The product was shown to have composition 9B.

## Example 10

The procedure of example 1 was followed except that aniline (8.69 g; 93 mmol) was applied instead of m-toluidine. A sample taken 2 minutes after complete addition of isopropyl chloride, was shown to have composition 10A. The product (11.85 g) after a reaction time of 45 minutes was shown to have composition 10B. 76.2% of the aniline is converted with a selectivity of 83% towards the para-compound after a reaction time of 45 minutes.

|  | 10A | 10B |
|---|---|---|
| aniline | 42.4 | 23.8 |
| o-isopropylaniline } m-isopropylaniline | 0.9 | 1.2 |
| p-isopropylaniline | 54.0 | 63.5 |
| diisopropylaniline | 2.0 | 9.4 |

## Example 11

The procedure of example 1 was followed except that N,N-dimethyl-m-toluidine (12.62 g; 93 mmol) was applied instead of m-toluidine. A sample taken 2 minutes after complete addition of isopropyl chloride, was shown to have composition 11A. The product obtained after a reaction time of 45 minutes was shown to have a composition 11B. After 45 minutes both the conversion and the selectivity towards para-alkylation are 87%.

|  | 11A | 11B |
|---|---|---|
| unconverted product | 37.6 | 13.4 |
| o-alkylated product } m-alkylated product | 6.1 | 8.0 |
| p-alkylated product | 54.1 | 75.6 |
| dialkylated product | — | — |

## Example 12

The procedure of example 1 was followed except that n-butyl bromide (14.95 g; 109 mmol) was applied as the alkylating agent. The temperature temporarily rose to about 10.5°C. A sample taken 2 minutes after complete addition of the alkylating agent, was shown to have composition 12A. Because of the low conversion rate, the reaction was allowed to proceed for 3 hours. The product (11.5 g) was shown to have composition 12B. The alkylated product was shown to be 3-methyl-4-sec.butylaniline.

|  | 12A | 12B |
|---|---|---|
| unconverted product | 84.4 | 53.9 |
| o-alkylated product | — | 1.2 |
| m-alkylated product | — | 1.6 |
| p-alkylated product | 15.6 | 37.9 |
| dialkylated product | — | 1.6 |

## Example 13

The procedure of example 1 was followed except that N-methylaniline was used instead of m-toluidine. A sample taken 2 minutes after complete addition of isopropyl chloride had composition 13A. The product (after 37 minutes) had composition 13B.

|  | 13A | 13B |
|---|---|---|
| Unconverted product | 42.0 | 18.7 |
| o-alkylated product } m-alkylated product | 1.4 | 3.3 |
| p-alkylated product | 55.7 | 75.4 |
| dialkylated product | — | — |

## Example 14

The procedure of example 1 was followed except that 2-fluoroaniline was used instead of m-toluidine, and the reaction temperature was 50°C. After 2 minutes, 36.9% of the starting material had been converted to product, 79.4% of which consisted of 2-fluoro-4-isopropylaniline. After 37 minutes, 45.6% of the starting material had been converted to products, 70.7% of which consisted of 2-fluoro-4-isopropylaniline.

**Claims**

1. Process for the selective alkylation (as hereinbefore defined) of an aniline containing an —N(R)$_2$ group in which each R independently represents a hydrogen atom or an alkyl group, by reacting the aniline with an alkylating agent in the presence of a molar excess of an aluminium halide, characterised in that the aniline is selectively alkylated at the nulcear position para to the amino group by reacting it in a polar organic solvent at a temperature in the range from −30° to +40°C in the presence of at least 1.02 mole of aluminium halide per mole of the aniline.

2. Process as claimed in claim 1, characterised in that the solvent is 1,2-dichloroethane.

3. Process as claimed in claim 1 or 2, characterised in that the alkylation is carried out at a temperature in the range of from −5° to −20°C.

4. Process as claimed in claim 1, 2 or 3, characterised in that the aluminium halide is present in an amount of form 1.05 to 1.15 mole per mole of the aniline.

5. Process as claimed in any one of the preceding claims, characterised in that the alkylating agent is isopropyl chloride.

6. Process as claimed in any one of the preceding claims, characterised in that the molar ratio of the alkylating agent to the aniline lies in the range of from 0.5:1 to 1.3:1.

**Patentansprüche**

1. Verfahren zum selektiven Alkylieren (wie zuvor definiert) eines eine —N(R)$_2$ -Gruppe enthaltenden Anilins, wobei jedes R unabhängig voneinander ein Wasserstoffatom oder eine Alkylgruppe darstellts, durch Umsetzen des Anilins mit einem Alkylierungsmittel in Gegenwart eines molaren Überschusses eines Aluminiumhalogenids, dadurch gekennzeichnet, daß das Anilin selektiv am Kern in p-Stellung zur Aminogruppe alkyliert wird, indem es in einem polaren organischen Lösungsmittel bei einer Temperatur im Bereich von −30° bis +40°C in Gegenwart von mindestens 1,02 Mol Aluminiumhalogenid je Mol Anilin umgesetzt wird.

2. Verfahren wie in Anspruch 1 beansprucht, dadurch gekennzeichnet, daß das Lösungsmittel 1,2-Dichloräthan ist.

3. Verfahren wie in Anspruch 1 oder 2 beansprucht, dadurch gekennzeichnet, daß die Alkylierung bei einer Temperatur im Bereich von −5°C bis +20°C durchgeführt wird.

4. Verfahren wie in Anspruch 1, 2 oder 3 beansprucht, dadurch gekennzeichnet, daß das Aluminiumhalogenid in einer Menge von 1,05 bis 1,15 Mol je Mol Anilin vorliegt.

5. Verfahren wie in irdendeinem der vorstehenden Ansprüche beansprucht, dadurch gekennzeichnet, daß das Alkylierungsmittel Isopropylchlorid ist.

6. Verfahren wie in irgendeinem der vorstehenden Ansprüche beansprucht, dadurch gekennzeichnet, daß das molare Verhältnis des Alkylierungsmittels zu dem Anilin im Bereich von 0,5:1 bis 1,3:1 liegt.

**Revendications**

1. Procédé pour l'alkylation sélective (telle que définie précédemment) d'une aniline contenant un groupe —N(R)$_2$ dans lequel chaque R représente, indépendamment, un atome d'hydrogène ou un groupe alkyle, par réaction de l'aniline avec un agent d'alkylation en présence d'un excès molaire d'un halogénure d'aluminium, caractérisé en ce que l'aniline est sélectivement alkylée en position para du noyau par rapport au groupe amino par réaction de celle-ci dans un solvant polaire à une température comprise dans la gamme de −30° à +40°C en présence d'au moins 1,02 moles d'halogénure d'aluminium par mole d'aniline.

2. Procédé selon la revendication 1, caractérisé en ce que le solvant est le dichloréthane-1,2.

3. Procédé selon la revendication 1 ou 2, caractérisé en ce que l'alkylation s'effectue à une température comprise dans la gamme de −5° à +20°C.

4. Procédé selon la revendication 1, 2 ou 3, caractérisé en ce que l'halogénure d'aluminium est présente à raison de 1,05 à 1,15 moles per mole d'aniline.

5. Procédé selon l'une quelconque des revendications précédentes, caractérisé en ce que l'agent d'alkylation est le chlorure d'isopropyle.

6. Procédé selon l'une quelconque des revendications précédentes, caractérisé en ce que le rapport molaire de l'agent d'alkylation à l'aniline se situe dans la gamme de 0,5:1 à 1,3:1.